# EUROPEAN PATENT APPLICATION

(11) **EP 1 964 920 A1**
(43) Date of publication of application: **03.09.2008**
(21) Application number: 08003130.5
(22) Date of filing: 20.02.2008
(51) Int. Cl.: C12N 15/10, C12Q 1/68

(54) **Instrument and method for nucleic acid isolation**

(30) Priority: 01.03.2007 JP 2007052037
(71) Applicant: Hitachi High-Technologies Corporation, Tokyo 105-8717 (JP)
(72) Inventor: Yamashita, Yoshihiro, Hitachinaka-shi Ibaraki 312-8504 (JP); Sakurai, Toshinari, Hitachinaka-shi Ibaraki 312-8504 (JP)
(74) Representative: Strehl Schübel-Hopf & Partner

(57) **Abstract**

According to the present invention, purified nucleic acids are isolated without the complete removal of components of a washing reagent.
Also, nucleic acids are isolated in an elution reagent by allowing a washing reagent comprising at least one component constituting a reaction solution that can be applied to a reaction involving nucleic acids to come into contact with a nucleic-acid-adsorbing solid phase so as to wash the solid phase, separating the washing reagent from the solid phase in a manner such that a certain amount of the washing reagent is allowed to remain in the solid phase, and allowing an elution reagent to come into contact with the nucleic-acid-adsorbing solid phase.

SEQUENCE LISTING

<110>  Hitachi High-Technologies Corporation

<120>  A method for collecting a nucleic acid and an apparatus thereof

<130>  A601828

<160>  6

<170>  PatentIn version 3.4

<210>  1
<211>  27
<212>  DNA
<213>  Artificial

<220>
<223>  synthetic DNA

<400>  1
gcggatccga gttactctcg tttttgc                                          27

<210>  2
<211>  29
<212>  DNA
<213>  Artificial

<220>
<223>  synthetic DNA

<400>  2
gcaagctttc taacaacagt agtttccgg                                        29

<210>  3
<211>  46
<212>  DNA
<213>  Artificial

<220>
<223>  synthetic DNA

<400> 3
cctgctgcta tgcctcatct tctttgacaa acgggcaaca tacctt 46

<210> 4
<211> 44
<212> DNA
<213> Artificial

<220>
<223> synthetic DNA

<400> 4
gataaaacgc cgcagacaca tccttccaac ctcttgtcct ccaa 44

<210> 5
<211> 19
<212> DNA
<213> Artificial

<220>
<223> synthetic DNA

<400> 5
ggtggttgat gttcctgga 19

<210> 6
<211> 20
<212> DNA
<213> Artificial

<220>
<223> synthetic DNA

<400> 6
caaaattcgc agtccccaac 20

## Description

### BACKGROUND OF THE INVENTION

### Field of the Invention

The present invention relates to an instrument and a method for nucleic acid isolation, wherein nucleic acids are eluted from a nucleic-acid-adsorbing solid phase and then isolated in a solution.

### Background Art

Analysis of nucleic acid, which is a substance that carries genetic information, is being carried out in a wide variety of fields, such as academic research and medicine. Nucleic acid analysis often involves nucleic acid amplification techniques, typically polymerase chain reaction (PCR). When performing a nucleic acid analysis such as PCR, it is necessary to isolate nucleic acids from a biological sample or the like and purify them to an extent such that the sample does not contain substances inhibiting analysis reaction.

In one conventional method for nucleic acid isolation from a biological sample, an operation such as ethanol precipitation or the like is carried out using poisonous material such as phenol or chloroform. In recent years, it has been common to use a method utilizing the property of nucleic acids to become adsorbed on a silica-containing solid phase or the like in the presence of a chaotropic agent.

For instance, the method described in J. Clin. Microbiol., 28(3), 495-503 (1990) comprises the following steps of: (1) allowing nucleic acids to become adsorbed on a solid phase by mixing a lysing solution containing a chaotropic agent and a silica-containing solid phase with a biological sample comprising nucleic acids; (2) washing the complex comprising nucleic acids and a solid phase with the use of a washing reagent containing a chaotropic agent; (3) washing the complex comprising nucleic acids and a solid phase with the use of a washing reagent containing ethanol; (4) washing the complex comprising nucleic acids and a solid phase with the use of a washing reagent containing acetone; (5) dehydrating the remaining washing reagent by heating the complex comprising nucleic acids and a solid phase; and (6) eluting nucleic acids from a silica-containing solid phase. In short, according to the above method, in order to wash a complex comprising nucleic acids and a solid phase, a washing reagent containing a chaotropic agent, a washing reagent containing ethanol, and a washing reagent containing acetone are used in such order, followed by dehydration and removal of the remaining washing reagent (mainly containing acetone) by heating the complex comprising nucleic acids and a solid phase.

However, according to the above method, it is necessary to use a plurality of washing reagents for washing of a complex comprising nucleic acids and a solid phase and also to carry out a step of heating dehydration. Thus, the method is highly complicated and time-consuming for obtaining purified nucleic acids, which is problematic. Further, in this method, a heating operation for promoting dehydration of an organic solvent is dangerous due to inflammability of the organic solvent, which is also problematic.

In addition, the method described in JP Patent Publication (Kokai) No. 11-146783 A (1999) comprises the following steps of: (1) allowing nucleic acids to become adsorbed on a solid phase by mixing a lysing solution containing a chaotropic agent and a silica-containing solid phase with a sample comprising ribonucleic acids; (2) washing the complex comprising nucleic acids and a solid phase with the use of a washing reagent containing a chaotropic agent; (3) washing the complex comprising nucleic acids and a solid phase with the use of a washing reagent containing water or a low salt concentration buffer; and (4) eluting nucleic acids from the silica-containing solid phase by allowing an elution reagent containing water or a low salt concentration buffer to come into contact with the complex comprising nucleic acids and a solid phase so as to heat the complex. According to the above method, a step of washing with the use of a washing reagent not containing an organic solvent such as ethanol is carried out. However, the method is based on the property of ribonucleic acids to become tightly adsorbed on a solid phase, and thus the method cannot be applied to deoxyribonucleic acid isolation, which is problematic. Also, with this method, the presence of a chaotropic agent carried over in a washing reagent is problematic.

Further, the method described in JP Patent Publication (Kokai) No. 11-127854 A (1999) comprises the following steps of: (1) promoting release of nucleic acids from a sample containing nucleic acids; (2) mixing a substance that promotes the adsorption of released nucleic acids on a solid phase; (3) allowing the nucleic acids to become adsorbed on the solid phase by allowing a liquid mixture to come into contact with the silica-containing solid phase; (4) separating a complex comprising nucleic acids and a solid phase from the liquid mixture; (5) washing the complex comprising nucleic acids and a solid phase with a solution containing a salt; and (6) eluting nucleic acids from the solid phase. However, in the above method, a washing reagent containing an organic solvent such as ethanol is used in the step (5). Thus, it is described that an operation for removing ethanol is necessary. Also, in this method, the amount of a salt contained in a purified nucleic acid solution is not examined, such salt having been originally contained in the washing reagent used in step (5).

### SUMMARY OF THE INVENTION

As described above, some techniques exist that are intended to completely remove components of a washing reagent when a step of washing a complex comprising nucleic acids and a solid phase so as to elute nucleic acids from the solid phase is carried out. However, it is difficult to completely remove components of a washing reagent, and such operation is very time-consuming. Thus, it is an objective of the present invention to provide a method and an instrument for nucleic acid isolation, whereby it is possible to isolate purified nucleic acids without completely removing components of a washing reagent.

The method for nucleic acid isolation of the present invention, whereby the above objective has been achieved, comprises:
allowing a washing reagent comprising at least one component constituting a reaction solution that can be applied to a reaction involving nucleic acids to come into contact with a nucleic-acid-adsorbing solid phase so as to wash the solid phase;
separating the washing reagent from the solid phase in a manner such that a certain amount of the washing reagent is allowed to remain in the solid phase; and
allowing an elution reagent to come into contact with the nucleic-acid-adsorbing solid phase so as to isolate nucleic acids in the elution reagent.

According to the method for nucleic acid isolation of the present invention, it is preferable that the elution reagent mixed with the washing reagent remaining in the solid phase have a composition that results in the reaction solution that can be applied to a reaction involving nucleic acids. Also, according to the method for nucleic acid isolation of the present invention, a component may be added in a manner such that the component mixed with the washing reagent remaining in the solid phase results in the reaction solution that can be applied to a reaction involving nucleic acids following elution of nucleic acids in the elution solution. In addition, the method for nucleic acid isolation of the present invention may further comprise a treatment of allowing the nucleic acids to become adsorbed on a solid phase by allowing a solution containing nucleic acids to come into contact with the solid phase.

Examples of components constituting the above reaction solution include a salt and/or a surfactant. When a nucleic acid amplification reaction is carried out with the use of an elution reagent containing nucleic acids according to the method for nucleic acid isolation of the present invention, the washing reagent used comprises at least one component selected from salt components and surfactant components contained in a reaction solution for the nucleic acid amplification reaction. Examples of a nucleic acid amplification reaction include a polymerase chain reaction (PCR) and the LAMP (loop-mediated isothermal amplification) method.

In addition, according to the method for nucleic acid isolation of the present invention, a certain amount of the washing reagent is allowed to remain by adjusting the amount of the above washing reagent to be added and the amount of the same to be discarded. Further, according to the method for nucleic acid isolation of the present invention, a certain amount of the washing reagent is allowed to remain by adjusting the moisture content of the solid phase and the moisture content of a solid-phase-supporting member that positions the solid phase. Furthermore, according to the method for nucleic acid isolation of the present invention, a certain amount of the washing reagent is allowed to remain by adjusting discharge pressure or centrifugal force upon separation of the washing reagent from the solid phase.

Meanwhile, the instrument for nucleic acid isolation of the present invention, whereby the above objective has been achieved, comprises:
a supply unit for supplying a washing reagent that supplies a washing reagent comprising at least one component constituting a reaction solution that can be applied to a reaction involving nucleic acids to a solid phase capable of adsorbing nucleic acids;
a separation unit for separating a washing reagent that separates the solution from the solid phase in a manner such that a certain amount of the washing reagent supplied to the solid phase is allowed to remain in the solid phase; and
a supply unit for supplying an elution reagent that supplies an elution reagent to the nucleic-acid-adsorbing solid phase following removal of the washing reagent by the separation unit for separating a washing reagent; wherein
nucleic acids are isolated in an elution reagent supplied by the supply unit for supplying an elution reagent.

According to the instrument for nucleic acid isolation of the present invention, a washing reagent supplied by a supply unit for supplying a washing reagent is allowed to come into contact with a nucleic-acid-adsorbing solid phase such that the solid phase is washed. Thereafter, the washing reagent is removed by a separation unit for separating a washing reagent in the instrument for nucleic acid isolation of the present invention. Upon removal, a certain amount of the washing reagent is allowed to remain in or in the vicinity of the solid phase. Then, in the instrument for nucleic acid isolation of the present invention, nucleic acids can be eluted from a solid phase by allowing an elution reagent supplied by a supply unit for supplying an elution reagent to come into contact with a nucleic-acid-adsorbing solid phase.

In the instrument for nucleic acid isolation of the present invention, the elution reagent mixed with the washing reagent remaining in the solid phase may have a composition that results in the reaction solution that can be applied to a reaction involving nucleic acids. In addition, the instrument for nucleic acid isolation of the present invention may have an addition unit for adding a component that adds a component in a manner such that the component mixed with the washing reagent remaining in the solid phase results in the reaction solution that can be applied to a reaction involving nucleic acids following elution of nucleic acids in the elution solution.

According to the instrument for nucleic acid isolation of the present invention, the above supply unit for supplying a washing reagent can fill a container that accommodates the solid phase with a washing reagent. The supply unit for supplying a washing reagent may be composed of a tank filled with a washing reagent and a dispenser capable of supplying a certain amount of a washing reagent contained in the tank to the above container, but it is not particularly limited thereto. In addition, the supply unit for supplying a washing reagent may be composed of a tank filled with a washing reagent and an aspiration instrument capable of aspirating a certain amount of a washing reagent into the container, but it is not particularly limited thereto.

According to the instrument for nucleic acid isolation of the present invention, the above separation unit for separating a washing reagent can remove a washing reagent with which the container that accommodates the solid phase is filled. The separation unit for separating a washing reagent may be composed of a dispenser capable of aspirating a certain amount of a washing solution supplied to the above solution for isolation, but it is not particularly limited thereto. The separation unit for separating a washing reagent may be a discharge instrument capable of discharging a certain amount of a washing reagent supplied to the above solution, but it is not particularly limited thereto.

In addition, the aspiration and discharge instruments exemplified above can be integrated into a pump instrument capable of aspirating and discharging a washing reagent by depressurizing and pressurizing the container that accommodates the solid phase. That is, according to the instrument for nucleic acid isolation of the present invention, the supply unit for supplying a washing reagent and the separation unit for separating a washing reagent described above can constitute a single instrument.

In addition, according to the instrument for nucleic acid isolation of the present invention, the above supply unit for supplying an elution reagent can fill the container that accommodates the solid phase with an elution reagent. The supply unit for supplying an elution reagent may be composed of a tank filled with an elution reagent and a dispenser capable of supplying a certain amount of an elution reagent contained in the tank to the container, but it is not particularly limited thereto. In addition, the supply unit for supplying an elution reagent may be composed of a tank filled with an elution reagent and an aspiration instrument capable of aspirating a certain amount of an elution reagent into the container, but it is not particularly limited thereto. Further, an aspiration instrument constituting a supply unit for supplying an elution reagent serves as a pump instrument capable of aspirating and discharging an elution reagent by depressurizing and pressurizing the container that accommodates a solid phase. Thus, the supply unit for supplying a washing reagent and the separation unit for separating a washing reagent described above can constitute a single instrument.

Further, according to the instrument for nucleic acid isolation of the present invention, the above container may comprise a solid-phase-supporting member capable of positioning a solid phase. In such case, in the instrument for nucleic acid isolation, a certain amount of a washing reagent supplied by the supply unit for supplying a washing reagent is allowed to remain in the solid-phase-supporting member and /or the solid phase. In such case, it is possible to adjust the amount of the washing reagent maintained depending on the materials and/or the volumes of the solid-phase-supporting member and the solid phase.

### Effects of the Invention

According to the present invention, it is possible to isolate nucleic acids in a solution having a desired composition by allowing an elution reagent to come into contact with a nucleic-acid-adsorbing solid phase upon elution of the nucleic acids. Thus, according to the present invention, an elution reagent can be applied to a reaction involving nucleic acids, such as a nucleic acid amplification reaction.

### BRIEF DESCRIPTION OF THE DRAWINGS

Fig. 1 is a flow chart of an example of a method for nucleic acid isolation to which the present invention is applied.
Fig. 2 is a flow chart of another example of a method for nucleic acid isolation to which the present invention is applied.
Fig. 3 is a flow chart of another example of a method for nucleic acid isolation to which the present invention is applied.
Fig. 4 is a cross-section showing an example of an instrument for nucleic acid isolation that constitutes an instrument for nucleic acid isolation to which the present invention is applied.
Fig. 5 is a cross-section showing another example of an instrument for nucleic acid isolation that constitutes an instrument for nucleic acid isolation to which the present invention is applied.
Fig. 6 is a characteristic diagram showing results of Example 1.

### DESCRIPTION OF THE PREFERRED EMBODIMENTS

Hereafter, the present invention will be described in detail with reference to the drawings.

In the method for nucleic acid isolation of the present invention, a washing reagent comprising at least one component constituting a reaction solution that can be applied to a reaction involving nucleic acids is first allowed to come into contact with a nucleic-acid-adsorbing solid phase such that the solid phase is washed. The term "nucleic-acid-adsorbing solid phase" used herein indicates, but is not particularly limited to, a solid phase capable of adsorbing nucleic acids, on which nucleic acids have been adsorbed. In addition, the adsorption of nucleic acids on a solid phase includes any form of chemical binding such as covalent binding, ionic binding, or Van der Waals binding. Examples of a solid phase include, but are not particularly limited to, a silica-containing solid phase made of glass, silica, diatomaceous earth, or the like or a solid phase comprising organic substances such as acetylcellulose. A solid phase can be formed by processing such materials into particulates or a porous membrane.

In addition, nucleic acids can be obtained by cultured cells, tissue sections obtained from a variety of tissues, microorganisms such as bacteria, fungi, and archaebacteria that have been cultured in a certain medium, and biological materials such as plant cells, plant tissue sections, insect cells, and insects according to a conventional method. Such nucleic acids are obtained in the form of, for example, a suspension further containing non-nucleic acid components such as proteins, lipids, and cell-wall components. When nucleic acids are allowed to become adsorbed on a solid phase, such nucleic acids can be applied to a method utilizing the property of nucleic acids to become adsorbed on a solid phase in the presence of a chaotropic agent, for example. Examples of a chaotropic agent that can be used include sodium iodide, potassium iodide, sodium perchlorate, sodium thiocyanate, guanidine thiocyanate, and guanidine hydrochloride.

For instance, when solid-phase particulates are used, a chaotropic agent and solid-phase particulates are added to a suspension containing nucleic acids, followed by agitation. Accordingly, nucleic acids contained in the suspension become adsorbed on solid-phase particulates.

Further, when a solid phase in the form of a porous membrane is used, an instrument in which a solid phase in the form of a porous membrane is fixed inside a hollow tube is used. In addition, a solid phase in the form of a porous membrane can be fixed inside such tube in a manner such that the solid phase is sandwiched by a pair of solid-phase-supporting members. In such case, a pair of solid-phase-supporting members can be formed with, for example, porous sintered polypropylene particles. When such instrument is used, one end of a tube is opened, the other end is connected to a pump instrument, and the pump instrument is driven, such that a solution can be aspirated and discharged through the opened end. Accordingly, the solution is allowed to come into contact with the solid phase in the form of a porous membrane. Alternatively, the solution aspirated into the instrument can be discharged with the use of centrifugal force.

According to the method for nucleic acid isolation of the present invention, a washing reagent is allowed to come into contact with a nucleic-acid-adsorbing solid phase that is prepared as described above. The washing reagent used is defined as a washing reagent comprising at least one component constituting a reaction solution that can be applied to a reaction involving nucleic acids. Examples of a reaction involving nucleic acids exemplified herein include: enzymatic reactions such as a nucleic acid amplification reaction, a reverse transcription reaction, a nucleic acid elongation reaction, and a restriction enzyme reaction; and electrophoresis. Further specific examples of a reaction involving nucleic acids exemplified herein include PCR (polymerase chain reaction), RT-PCR (reverse transcription-polymerase chain reaction), LCR (ligase chain reaction), SDA (strand displacement amplification), TAS (transcription amplification system), NASBA (nucleic acid sequence-based amplification), TMA (transcription-mediated amplification), LAMP (loop-mediated isothermal amplification), and ICAN (isothermal and chimeric primer-initiated amplification of nucleic acids).

Reaction solutions that can be applied to the above reactions are solutions containing a buffering agent used for pH maintenance at a predetermined level, an inorganic salt at a predetermined concentration, a surfactant at a predetermined concentration, a predetermined enzyme, and the like. In addition, such reaction solutions have different compositions so as to be used for different reactions described above. The composition and the composition ratio of each reaction solution can be unambiguously determined with reference to the corresponding description (PCR (Saiki R. K. et al. (1985), Science, vol. 230, 1350-1354); PCR and RT-PCR (Molecular Cloning, a laboratory manual (third edition), chapter 8.1); LCR (Landegren U. et al. (1988), Science, vol. 239, 487-491); SDA (Walker G. T. et al. (1992), Proc. Natl. Acad. Sci. USA, vol. 89, 392-396); TAS (Kwoh D. Y. et al. (1989), Proc. Natl. Acad. Sci. USA, vol. 86, 1173-1177); NASBA (Compton J. (1991), Nature, vol. 350. 91-92); TMA (JP Patent No. 3241717); LAMP (Notomi T. et al. (2000), Nucleic Acid Res., vol. 28, e63); and ICAN (WO2000-56877)). Thus, by referring to the above descriptions, the composition of a washing reagent comprising at least one component constituting a reaction solution can be determined.

In such case, examples of a washing reagent that can be used include a washing reagent containing components such as a salt (inorganic salt) and a surfactant that constitute a reaction solution. Preferably, a salt and a surfactant have concentrations at which the adsorption of nucleic acids on a solid phase is maintained and impurities (e.g., a chaotropic agent) remaining in the solid phase are removed. Preferably, a washing reagent contains a salt at 0.5 M or more, more preferably at 1.0 M or more, and most preferably at 1.5 M or more. When the salt concentration of a washing reagent is less than 0.5 M, nucleic acids might be eluted from a solid phase upon washing. As a result, the yield of nucleic acids decreases in some cases.

In addition, upon washing with the use of a washing reagent, it is preferable to carry out washing at low temperatures in order to maintain the adsorption of nucleic acids on a solid phase. The temperature upon washing (temperature of a washing reagent when mixed with a solid phase) is preferably 40°C or less, more preferably 20°C or less, and most preferably 10°C or less. When the temperature upon washing exceeds 40°C, nucleic acids might be eluted from a solid phase upon washing. As a result, the yield of nucleic acids decreases in some cases.

For instance, in the case of the use of a PCR reaction solution as a reaction solution, a standard PCR reaction solution comprises, as components, a reaction buffer (10 mM Tris-HCl (pH 8.8), 50 mM KCl, and 1.5 mM MgCl₂), a 0.8 mM dNTP mix (dATP, dCTP, dGTP, and dTTP), 0.5 µM Primer 1, 0.5 µM Primer 2, 2.5 U Taq DNA Polymerase, and analyte DNA. For instance, a washing reagent comprises, as a component that can be used, a solution at a salt concentration 50 times higher than the salt concentration in a reaction buffer (i.e., a solution comprising 2.5 M KCl and 75 mM MgCl₂). When a solution comprising 2.5 M KCl and 75 mM MgCl₂ is used as a washing reagent, it is possible to maintain the adsorption of nucleic acids on a solid phase and to remove impurities remaining in the solid phase.

For instance, in the case of the use of a LAMP reaction solution as a reaction solution, a standard LAMP reaction solution comprises, as components, a reaction buffer (10m M KCl, 20 mM Tris-HCl (pH 8.8), 10 mM (NH₄)₂SO₄, 4 mM MgSO₄, and 0.1% TritonX-100), 1M Betaine, a 1.6 mM dNTP mix (dATP, dCTP, dGTP, and dTTP), 0.8 µM Primer FIP, 0.8 µM Primer BIP, 0.2 µM Primer F3, 0.2 µM Primer B3, 8U Bst DNA Polymerase, and analyte DNA. For instance, a washing reagent comprises, as a component that can be used, a solution at a salt concentration 50 times higher than the salt concentration in a reaction buffer (i.e., a solution comprising 500 mM KCl, 500 mM (NH₄)₂SO₄, and 200 mM MgSO₄). When a solution comprising 500 mM KCl, 500 mM (NH₄)₂SO₄, and 200 mM MgSO₄ is used as a washing reagent, it is possible to maintain the adsorption of nucleic acids on a solid phase and to remove impurities remaining in the solid phase.

Further, for instance, in the case of the use of a restriction enzyme reaction solution as a reaction solution, a standard reaction solution, which is used in a restriction enzyme reaction caused by restriction enzyme *Hind*III*,* comprises, as components, a reaction buffer comprising 10mM Tris-HCl (pH 8.5), 10mM MgCl₂, 100 mM NaCl, and 10 mM 2-Mercaptoethanol, 1U *Hind*III, and analyte DNA. For instance, a washing reagent comprises, as a component that can be used, a solution at a salt concentration 50 times higher than the salt concentration in a reaction buffer (i.e., a solution comprising 500 mM MgCl₂ and 5M NaCl). When a solution comprising 500 mM MgCl₂ and 5M NaCl is used as a washing reagent, it is possible to maintain the adsorption of nucleic acids on a solid phase and to remove impurities remaining in the solid phase.

Further, for instance, in the case of an electrophoresis reaction solution used as a reaction solution, a sample solution subjected to RNA electrophoresis with the use of a standard MOPS buffer comprises, as components, 20 mM MOPS, 5 mM CH₃COONa, 1 mM EDTA, 1 mM EGTA, 5% Glycerol, and analyte RNA. For instance, a washing reagent comprises, as a component that can be used, a solution containing components (other than glycerol) at concentrations 50 times higher than such concentrations in a reaction solution (i.e., a solution comprising 1 M MOPS, 250 mM CH₃COONa, 50 mM EDTA, and 50 mM EGTA). When a solution comprising 1M MOPS, 250 mM CH₃COONa, 50 mM EDTA, and 50 mM EGTA is used as a washing reagent, it is possible to maintain the adsorption of nucleic acids on a solid phase and to remove impurities remaining in the solid phase.

According to the method for nucleic acid isolation of the present invention, subsequently, a washing reagent is separated from a solid phase in a manner such that a certain amount of the washing reagent is allowed to remain in the solid phase. The washing reagent remaining in this step contains a component of the above reaction solution so that it can be used as a part of the reaction solution. In other words, according to the method for nucleic acid isolation of the present invention, a washing reagent used for removal of impurities, which remains in a solid phase, can be directly used for a reaction solution used in the subsequent step. Thus, it is not necessary to carry out a treatment or an operation to completely remove a washing reagent.

In order to remove a washing reagent, a dispenser, a pump instrument, and a centrifuge can be adequately used, but such items are not particularly limited thereto. When solid-phase particulates are used, washing is carried out by agitating the above washing reagent and the nucleic-acid-adsorbing solid phase in a container. Thereafter, a certain amount of the washing reagent is removed from the container by a dispensing means. As a result, the washing reagent can be removed from the solid phase in a manner such that a certain amount of the washing reagent is allowed to remain in the solid phase. In addition, when a solid phase in the form of a porous membrane is fixed inside a tube in a manner such that the solid phase is sandwiched by a pair of solid-phase-supporting members, the solid phase is washed by repeating aspiration and discharge with the use of a washing reagent. Then, the washing reagent is discharged such that a certain amount of the washing reagent is allowed to remain in the tube. In such case, it is also possible to allow the pair of solid-phase-supporting members to retain the washing reagent. Thus, even if the entirety of the liquid in the tube is removed, it is possible to allow a portion of the washing reagent, which is retained in the solid-phase-supporting members, to remain as is.

The remaining amount of a washing reagent may be adequately determined in consideration of the use of the remaining washing reagent as a part of a reaction solution. Such amount is not particularly limited. The remaining washing reagent is diluted with an elution reagent described below in detail or with such elution reagent and a solution added as a non-elution reagent. The resultant becomes a part of a reaction solution that can be applied to a reaction involving nucleic acids. Therefore, the remaining amount of a washing reagent can be determined based on the concentrations of components contained in the washing reagent and the amounts of an elution reagent and/or a solution added as a non-elution reagent. In other words, in order to obtain a reaction solution that is diluted so as to contain components at desired concentrations, the remaining amount of a washing reagent can be determined based on the concentrations of components contained in the washing reagent and the amounts of an elution reagent and/or a solution added as non-elution reagents.

In order to control the remaining amount of a washing reagent, for example, the amount of the washing reagent to be added and the amount of the same to be discarded are controlled such that a certain amount of the remaining washing reagent can be obtained. Alternatively, when a solid phase in the form of a porous membrane is fixed inside a tube in a manner such that the solid phase is sandwiched by a pair of solid-phase-supporting members, it is also possible to control the remaining amount of a washing reagent by adjusting the moisture content of the solid phase and the moisture content of the solid-phase-supporting members. For instance, the moisture content of a glass fiber filter that can be used in the form of a solid phase depends on volume, materials, fiber diameter, particle holding capacity, and the like. In addition, the moisture content of glass particles that can be used in the form of a solid phase depends on number of particles, materials, particle size, and the like. Further, the moisture content of porous sintered polypropylene particles that can be used in the form of a solid-phase-supporting member depends on volume, materials, particle size, particle holding capacity, and the like. Furthermore, the moisture content of a solid phase and the moisture content of solid-phase-supporting members depend on the method used for discarding the washing reagent (e.g., discharging pressure or centrifugal force when a washing reagent is discarded). Thus, the moisture content of a solid phase and the moisture content of solid-phase-supporting members can be controlled by adjusting physical properties of a solid phase and solid-phase-supporting members and the method used for discarding the washing reagent.

Next, according to the method for nucleic acid isolation of the present invention, an elution reagent is allowed to come into contact with a nucleic-acid-adsorbing solid phase such that nucleic acids are isolated in the elution reagent. The elution reagent used herein is not particularly limited as long as it functions to remove nucleic acids adsorbed on a solid phase from the solid phase so as to contain the nucleic acids. Examples of such elution reagent include water and Tris buffer. In addition, another example of the elution reagent that can be used is a solution comprising components contained in a washing reagent remaining in a solid phase and components constituting the composition of a reaction solution described below. That is, the composition of an elution reagent can be determined in a manner such that the elution reagent comprises components contained in the predetermined composition of a reaction solution (excluding components contained in a washing reagent) as long as such elution reagent can cause nucleic acids to be eluted from a solid phase. In addition, an elution reagent may have a composition comprising all the components contained in the predetermined composition of a reaction solution (excluding components contained in a washing reagent) or some of such components.

In addition, an elution reagent is preferably used in an amount at which dilution can be carried out in a manner such that components constituting a reaction solution, which are contained in a washing reagent remaining in a solid phase or the like, have predetermined concentrations in the reaction solution. Specifically, when a washing reagent contains components constituting a reaction solution at concentrations 50 times higher than predetermined concentrations in the reaction solution, it is preferable to use an elution reagent in an amount at which the remaining washing reagent is diluted 50-fold. As a result, components contained in a washing reagent are contained in a solution comprising a washing reagent and an elution reagent at predetermined concentrations in a reaction solution. In such case, if an elution reagent contains all the components contained in the predetermined composition of a reaction solution (excluding components contained in a washing reagent), a solution comprising a washing reagent and an elution reagent can be directly used as a reaction solution. In a case in which an elution reagent contains some of such components contained in the predetermined composition of a reaction solution, a reaction solution can be prepared by subsequently adding components that are not contained in the composition of a reaction solution to a solution comprising a washing reagent and an elution reagent. In such case, when components that are not contained in the composition of a reaction are added, a solution comprising a washing reagent and an elution reagent is diluted. Thus, it is necessary to check the concentrations of components contained in a washing reagent and an elution reagent.

For instance, in the case of the use of a PCR reaction solution as a reaction solution, a standard PCR reaction solution comprises, as components, a reaction buffer (10 mM Tris-HCl (pH 8.8), 50 mM KCl, and 1.5 mM MgCl₂), a 0.8 mM dNTP mix (dATP, dCTP, dGTP, and dTTP), 0.5 µM Primer 1, 0.5 µM Primer 2, 2.5 U Taq DNA Polymerase, and analyte DNA. For instance, when a solution at a salt concentration 50 times higher than the salt concentration in a reaction solution (i.e., a solution comprising 2.5 M KCl and 75 mM MgCl₂) is used as a washing reagent, an elution reagent that can be used is a solution comprising 10 mM Tris-HCl (pH 8.8), a 0.8 mM dNTP mix (dATP, dCTP, dGTP, and dTTP), 0.5 µM Primer 1, 0.5 µM Primer 2, and 2.5U Taq DNA Polymerase. In addition, an elution reagent may comprise such components at the above concentrations when mixed with a washing reagent. Further, such elution reagent is used in an amount at which the washing reagent is diluted 50-fold. As a result, the solution obtained after elution of nucleic acids from a solid phase with the use of an elution reagent contains eluted nucleic acids (to be used as PCR templates) and the complete composition of the PCR reaction solution, and thus it can be directly subjected to a PCR reaction.

In addition, in the case of the aforementioned LAMP reaction solution, restriction enzyme reaction solution, or electrophoresis reaction solution, the composition and the amount of an elution reagent can be determined in a similar manner. An elution reagent may have a composition not containing an enzyme alone in order to prevent enzyme deactivation due to temperatures. In such case, a reaction solution with a predetermined composition can be obtained by adding an enzyme as a remaining component to a solution comprising a washing reagent and an elution reagent.

Further, the temperature upon elution is not particularly limited. However, it is preferably 20°C or more, more preferably 40°C or more, and most preferably 60°C or more. When the temperature upon elution is less than 20°C, nucleic acids might be unable to be completely isolated from a solid phase or it might take long time to completely elute nucleic acid from a solid phase, which is problematic.

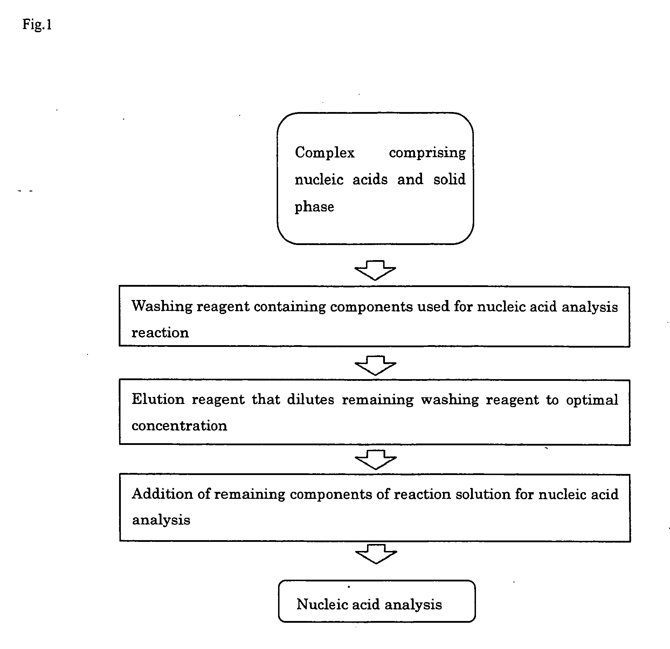

Figs. 1 to 3 are specific flow charts of the method for nucleic acid isolation of the present invention described above. In addition, the flow charts shown in figs. 1 to 3 are examples of the method for nucleic acid isolation of the present invention. Thus, the technical scope of the present invention is not limited thereto.

According to the flow chart shown in fig. 1, for example, the method for nucleic acid isolation of the present invention comprises the following steps of: (1) washing by allowing a washing reagent comprising components contained in a reaction solution for nucleic acid analysis described below to come into contact with a complex comprising nucleic acids and a solid phase; (2) eluting nucleic acids by allowing an elution reagent in an amount at which components that are contained in the remaining washing reagent obtained in the previous step are able to be diluted to concentrations appropriate for the subsequent nucleic acid analysis reaction to come into contact with the complex; and (3) adding remaining components of the reaction solution for nucleic acid analysis. In addition, the resulting solution is subjected to nucleic acid isolation.

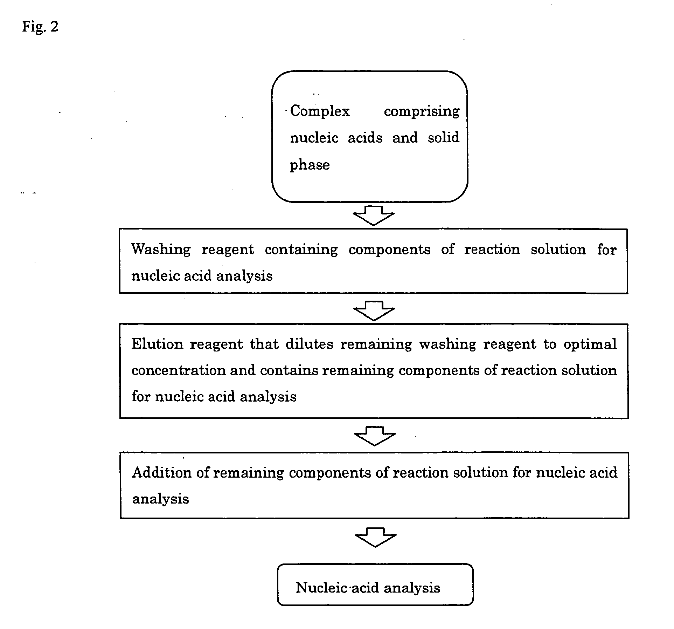

Further, according to the flow chart shown in fig. 2, for example, the method for nucleic acid isolation of the present invention comprises the following steps of : (1) washing by allowing a washing reagent comprising components contained in a reaction solution for nucleic acid analysis described below to come into contact with a complex comprising nucleic acids and a solid phase; (2) eluting nucleic acids by allowing an elution reagent in an amount at which components that are contained in the remaining washing reagent obtained in the previous step are able to be diluted to concentrations appropriate for the subsequent nucleic acid analysis reaction, such elution reagent comprising some other components necessary for a nucleic acid analysis reaction, to come into contact with the complex; and (3) adding remaining components of the reaction solution for nucleic acid analysis. In addition, the resulting solution is subjected to nucleic acid isolation.

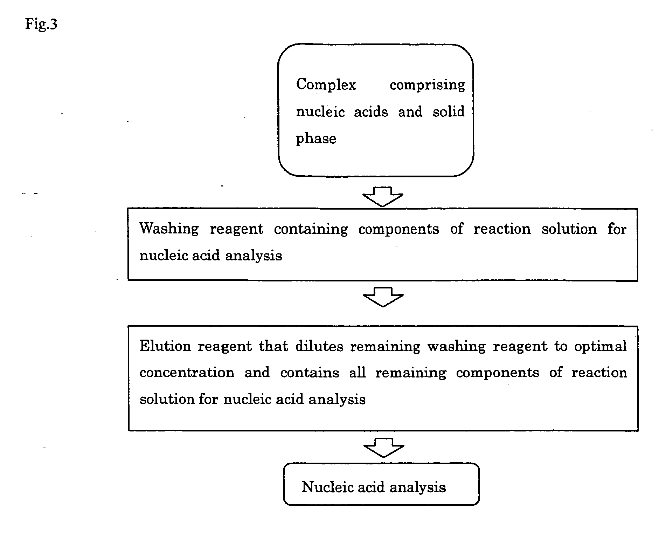

Furthermore, according to the flow chart shown in fig. 3, for example, the method for nucleic acid isolation of the present invention comprises the following steps of: (1) washing by allowing a washing reagent comprising components contained in a reaction solution for nucleic acid analysis described below to come into contact with a complex comprising nucleic acids and a solid phase; and (2) eluting nucleic acids by allowing an elution reagent in an amount at which components contained in the remaining washing reagent obtained in the previous step are able to be diluted to a concentration appropriate for the subsequent nucleic acid analysis reaction, such elution reagent comprising all the other components necessary for a nucleic acid analysis reaction, to come into contact with the complex. In addition, the resulting solution is subjected to nucleic acid isolation.

### [Examples]

The method and instrument for nucleic acid isolation of the present invention are hereafter described in greater detail with reference to the following examples, although the technical scope of the present invention is not limited thereto.

First, samples, reagents, instruments for nucleic acid isolation, a method for nucleic acid isolation, a method for nucleic acid quantification, and a method for nucleic acid analysis used in the Examples will be described below in order.

### 1. Samples

### Sample A:

A sample obtained by adding pBR322 plasmid DNA to reference serum (1 ug / 200 µl)

### Sample B:

A sample obtained by adding purified DNA derived from an HBV-positive specimen to reference serum (10³ copies / 200 µl)

### 2. Reagents

### Lysing solution A:

4.0 M guanidine thiocyanate
100 mM MES
25 mM EDTA-2Na
20% (v/v) TritonX-100

### First washing reagent:

4.0 M guanidine thiocyanate
100 mM MES
25 mM EDTA-2Na
1% (v/v) TritonX-100

### Second washing reagent:

10 mM Tris-HCl (pH 8.0)
80% (v/v) EtOH

### Third washing reagent A:

10 mM Tris-HCl (pH 8.0)
80% (v/v) EtOH

### Third washing reagent B:

2.5 M KCl
75 mM MgCl₂

### Third washing reagent C:

2.5 M KCl
75 mM MgCl₂
500 mM Tris-HCl (pH 8.0)

### Third washing reagent D:

2.5 M KCl
75 mM MgCl₂
500 mM Tris-HCl (pH 8.0)
0.5% TritonX-100

### Third washing reagent E:

500 mM KCl
500 mM (NH₄)₂SO₄
200 mM MgSO₄

### Third washing reagent F:

500 mM KCl
500 mM (NH₄)₂SO₄
200 mM MgSO₄
1 M Tris-HCl (pH 8.0)

### Third washing reagent G:

500 mM KCl
500 mM (NH₄)₂SO₄
200 mM MgSO₄
1M Tris-HCl (pH 8.0)
5% TritonX-100

### Elution reagent A:

10 mM Tris-HCl (pH 8.8)

### Elution reagent B:

Ultrapure water

### Elution reagent C:

10 mM Tris-HCl (pH 8.8)
0.8 mM dNTP mix (dATP, dCTP, dGTP, and dTTP)
0.5 µM Primer 1
0.5 µM Primer 2
2.5 U Taq DNA Polymerase

### Elution reagent D:

20 mM Tris-HCl (pH 8.8)
1 M Betaine
1.6 mM dNTP mix (dATP, dCTP, dGTP, and dTTP)
0.8 µM Primer FIP
0.8 µM Primer BIP
0.2 µM Primer F3
0.2 µM Primer B3
8 U Bst DNA Polymerase

### 3. Instrument for nucleic acid isolation

### Instrument for nucleic acid isolation A:

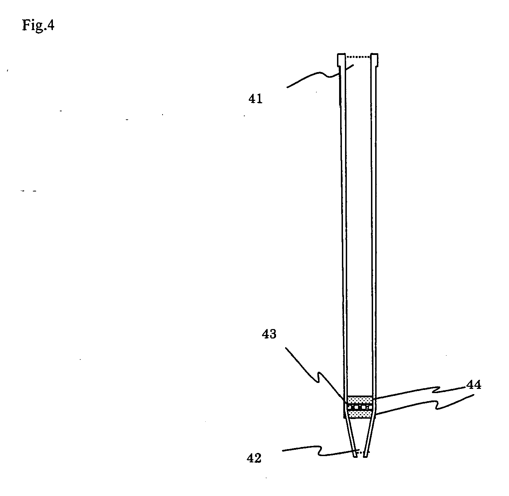

Fig. 4 shows a cross-section of an instrument for nucleic acid isolation A. As shown in fig. 4, the instrument for nucleic acid isolation A is composed of a pipette body 40 obtained by molding polypropylene, a pressurizing and depressurizing device (not shown) connected to one end 41 (opened) of the pipette body 40, a solid phase 43 that is disposed in the vicinity of the other end 42 (opened) of the pipette body 40, and solid-phase-supporting members 44 that position the solid phase 43 at a certain position in the pipette body 40. Such instrument for nucleic acid isolation A can aspirate and discharge a solution from the other end 42 due to the pressure difference generated by a pressurizing and depressurizing device. A solution aspirated into the pipette body 40 from the other end 42 is allowed to permeate through the solid phase 43 and the solid-phase-supporting members 44.

In addition, the solid phase 43 is a glass fiber filter and the solid-phase-supporting members 44 comprise porous sintered polypropylene particles.

### Instrument for nucleic acid isolation B:

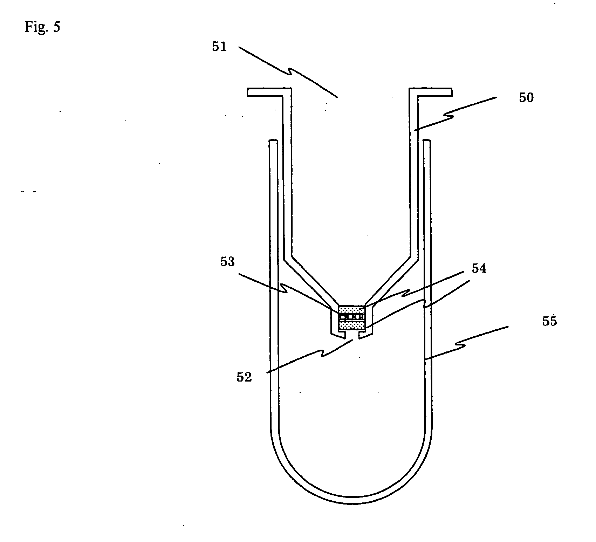

Fig. 5 shows a cross-section of an instrument for nucleic acid isolation B. As shown in fig. 5, the instrument for nucleic acid isolation B is composed of a container body 50, a solid phase 53 that is disposed in the vicinity of the other end 52 located opposite to one end 51 (opened upward) of the container body 50, solid-phase-supporting members 54 that position the solid phase 53, and a drip container 55 having an opening through which the container body 50 is inserted. In the instrument for nucleic acid isolation B, a solution is provided from the one end 51 of the container body 50. The solution provided to the container body 50 arrives at the solid phase 53 and the solid-phase-supporting members 54 so as to come into contact therewith. In such state, centrifugal force is applied to the instrument for nucleic acid isolation B in a direction from the one end 51 of the container body 50 to the bottom portion of the drip container 55. Accordingly, the solution provided to the container body 50 is allowed to be discharged into the drip container 55 from the other end 52 of the container body 50.

In addition, the solid phase 53 is a glass fiber filter and the solid-phase-supporting members 54 comprise porous sintered polypropylene particles.

### Instrument for nucleic acid isolation C:

An instrument for nucleic acid isolation C (not shown) comprises glass particles having an average particle size of 5 µm. The glass particles in the form of a solid phase are mixed with a solution in a given container and separated from a liquid phase by centrifugal force.

### 4. Method for nucleic acid isolation

A method for nucleic acid isolation with the use of the instrument for nucleic acid isolation A is described as follows. In addition, the following method for nucleic acid isolation can be applied regardless of types of samples, washing reagents, elution reagents, and the like.
(1) A lysing solution (0.8 ml) is added to a sample (0.2 ml), followed by mixing.
(2) A pressurizing and depressurizing device is connected to an instrument for nucleic acid isolation A, followed by aspiration and discharge of a liquid mixture 5 times.
(3) The liquid mixture is discarded.
(4) A first washing reagent (1.0 ml) is aspirated and discharged 3 times.
(5) The washing reagent is discarded.
(6) A second washing reagent (1.0 ml) is aspirated and discharged 3 times.
(7) The washing reagent is discarded.
(8) An ice-cooled third washing reagent (0.5 ml) is aspirated and discharged once.
(9) The washing reagent is discarded (volume of remaining liquid: 2 µl).
(10) An elution reagent (0.1 ml) heated at 60°C is aspirated and discharged 10 times.
(11) The elution reagent is isolated.

A method for nucleic acid isolation with the use of an instrument for nucleic acid isolation B is described as follows. In addition, the following method for nucleic acid isolation can be applied regardless of types of samples, washing reagents, elution reagents, and the like.
(1) A lysing solution (0.8 ml) is added to a sample (0.2 ml), followed by mixing.
(2) The mixture is introduced into an instrument for nucleic acid isolation B, followed by centrifugation.
(3) The liquid mixture in a drip container is discarded.
(4) A first washing reagent (1.0 ml) is introduced thereinto, followed by centrifugation.
(5) The washing reagent in the drip container is discarded.
(6) A second washing reagent (1.0 ml) is introduced thereinto, followed by centrifugation.
(7) The washing reagent in the drip container is discarded.
(8) An ice-cooled third washing reagent (0.5 ml) is introduced thereinto, followed by centrifugation.
(9) A new drip container is introduced (volume of remaining liquid: 2 µl).
(10) An elution reagent (0.1 ml) heated at 60°C is introduced thereinto, followed by centrifugation.
(11) The elution reagent is isolated.

A method for nucleic acid isolation with the use of an instrument for nucleic acid isolation C is described as follows. In addition, the following method for nucleic acid isolation can be applied regardless of types of samples, washing reagents, elution reagents, and the like.
(1) A lysing solution (0.8 ml) and glass particles (10 mg) are added to a sample (0.2 ml).
(2) Mixing is carried out for 10 minutes.
(3) B/F separation is carried out using a centrifuge and then the liquid phase is discarded.
(4) A first washing reagent (1.0 ml) is added to the sample, followed by mixing.
(5) B/F separation is carried out using a centrifuge, and then the liquid phase is discarded.
(6) A second washing reagent (1.0 ml) is added to the sample, followed by mixing.
(7) B/F separation is carried out using a centrifuge, and then the liquid phase is discarded.
(8) An ice-cooled third washing reagent (0.5 ml) is added to the sample, followed by mixing.
(9) B/F separation is carried out using a centrifuge and then the liquid phase is discarded (volume of remaining liquid: 2 µl).
(10) An elution reagent (0.1 ml) is added to the sample, followed by heating at 60°C for 5 minutes.
(11) B/F separation is carried out using a centrifuge such that the elution reagent is isolated.

### 5. Method for nucleic acid quantification

DNAs contained in a sample (nucleic acids contained in a washing reagent) were stained with a fluorescent dye specific to double-strand DNA and the fluorescence intensity was measured with a fluorometer for calculation of the DNA content. The fluorescent dye used was a PicoGreen dsDNA Quantitation Kit (Molecular Probe). The fluorometer used was an ARVO sx 1420 Multilabel Counter (Wallac).

### 6. Method for nucleic acid analysis

In this Example, a PCR method and the LAMP method were carried out as examples of a method for nucleic acid analysis in the following manner. In addition, amplification products obtained by a PCR method and the LAMP method were subjected to electrophoresis with the use of 0.8% agarose gel as a support and stained with a fluorescent dye specific to double-strand DNA, such that the products were confirmed using a transilluminator under ultraviolet irradiation. The fluorescent dye used was a SYBR Green I nucleic acid gel stain (Molecular Probe).

### PCR:

PCR with the use of HBV-derived DNA as a template DNA was carried out using the following primers under the following temperature conditions according to a report (J. Clin. Microbiol., 29(9), 1804-1811 (1991)).

Primer 1: 5'-GCG GAT CCG AGT TAC TCT CGT TTT TGC-3' (SEQ ID NO:
1)

Primer 2: 5'-GCA AGC TTT CTA ACA ACA GTA GTT TCC GG-3' (SEQ ID
NO: 2)

### Temperature conditions:

Retention at 94°C for 5 min., 30 cycles of 94°C for 1 min., 55°C for 1 min., and 72°C for 1 min., and retention at 72°C for 10 min.

### LAMP:

LAMP with the use of HBV-derived DNA as a template DNA was carried out using the following primers under the following temperature conditions according to a report (Nucleic Acid Research, 28(12), e63(2000)). 
Primer FIP: 5'-CCT GCT GCT ATG CCT CAT CTT CTT TGA CAA ACG
GGC AAC ATA CCT T-3' (SEQ ID NO: 3)
 
Primer BIP: 5'-GAT AAA ACG CCG CAG ACA CAT CCT TCC AAC CTC
TTG TCC TCC AA-3' (SEQ ID NO: 4)
 Primer F3: 5'-GGT GGT TGA TGT TCC TGG A-3' (SEQ ID NO: 5)
Primer B3: 5'-CAA AAT TCG CAG TCC CCA AC-3' (SEQ ID NO: 6)

### Temperature conditions:

Retention at 95°C for 5 min., placement on ice for 3 min., addition of Bst DNA Polymerase, a reaction at 65°C for 1 h., and retention at 80°C for 10 min.

### [Example 1]

DNA isolation was carried out with the use of a sample A, a lysing reagent A, a first washing reagent A, a second washing reagent A, an elution reagent A, and an instrument for nucleic acid isolation A. Upon DNA isolation, a diluted solution containing a third washing reagent A, B, or E (solute concentration: 100%, 80%, 60%, 40%, or 20%)) was also used as a third washing reagent. The DNA yield in each diluted solution comprising a third washing reagent A, B, or E was quantified. Then, the DNA yield percentage was calculated based on the DNA yield in each case of the use of the third washing reagent B or E relative to the DNA yield in the relevant case of the use of the third washing reagent A.

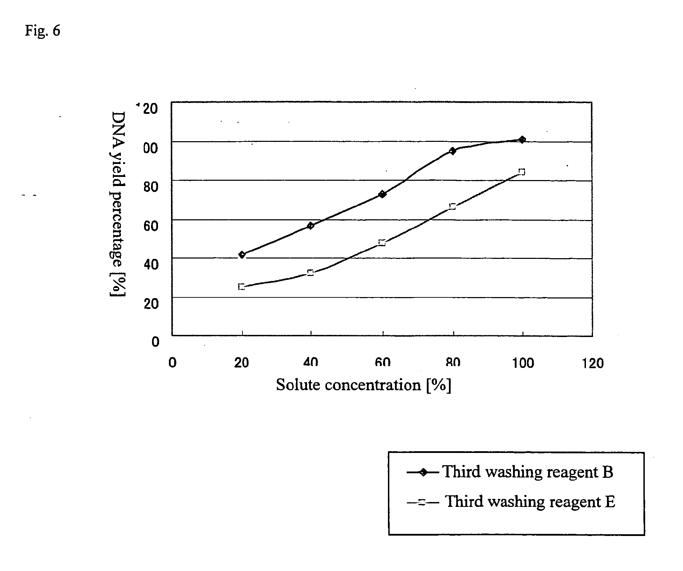

Fig. 6 shows the results. In the case of the third washing reagent B, the DNA yield percentage increased in line with an increase in the dilution percentage. At solute concentrations of 60% or more, the DNA yields became equivalent to those in the relevant cases corresponding to the third washing reagent A. Meanwhile, in the case of the third washing reagent E, the DNA yield percentage also increased in line with an increase in the dilution percentage. However, the DNA yield percentage was lower than that in the case of the third washing reagent B. It is considered that this is because the salt concentration of the third washing reagent E is lower than that of the third washing reagent B and the force of maintaining the adsorption of nucleic acids on a solid phase becomes small with the use of the third washing reagent E. Accordingly, it was shown that a washing reagent containing, at certain concentrations or higher, components that are necessary for a PCR reaction solution can maintain the adsorption of nucleic acids on a solid phase during a washing step, and thus a complex of nucleic acids and a solid phase can be washed with such washing reagent.

### [Example 2]

DNA isolation was carried out using a sample B, a lysing reagent A, a first washing reagent A, a second washing reagent A, and an instrument for nucleic acid isolation A. The table below lists third washing reagents, elution reagents, remaining components in a reaction solution for nucleic acid analysis, and nucleic acid analyses used. The occurrence or nonoccurrence of nucleic acid amplification in each nucleic acid analytical reaction was judged.

Experimental systems and results are summarized in table 1.

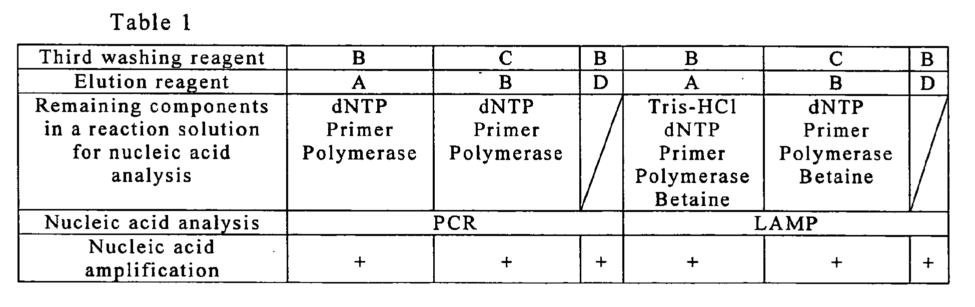

Based on the results listed in table 1, good nucleic acid amplification was confirmed for each combination of nucleic acid isolation and a nucleic acid amplification method. Accordingly, the results indicate that a nucleic acid solution that can be applied to a nucleic acid analysis reaction was obtained by carrying out washing with the use of a reagent containing components necessary for a nucleic acid amplification reaction and using a solution comprising an elution reagent and the remaining washing reagent as a reaction solution.

### [Example 3]

DNA isolation was carried out using a sample B, a lysing reagent A, a first washing reagent A, a second washing reagent A, a third washing reagent C, and an elution reagent B, followed by PCR. Upon DNA isolation, an instrument for nucleic acid isolation A, B, or C was used. Then, the occurrence or nonoccurrence of PCR amplification was judged. Table 2 shows the results.

**Table 2**

| Instrument for nucleic acid isolation | A | B | C |
|---|---|---|---|
| Nucleic acid amplification | + | + | + |

Based on the results listed in table 2, it was possible to confirm nucleic acid isolation and nucleic acid amplification in each case with the use of the instrument for nucleic acid isolation A, B, or C. Accordingly, it was shown that the method of the present invention can be applied to different methods for nucleic acid isolation utilizing the property of nucleic acids to become adsorbed on a solid phase.

## Claims

1. A method for nucleic acid isolation, comprising:
allowing a washing reagent comprising at least one component constituting a reaction solution that can be applied to a reaction involving nucleic acids to come into contact with a nucleic-acid-adsorbing solid phase so as to wash the solid phase;
separating the washing reagent from the solid phase in a manner such that a certain amount of the washing reagent is allowed to remain in the solid phase; and
allowing an elution reagent to come into contact with the nucleic-acid-adsorbing solid phase so as to isolate nucleic acids in the elution reagent.

2. The method for nucleic acid isolation according to claim 1, wherein the elution reagent mixed with the washing reagent remaining in the solid phase has a composition that results in the reaction solution that can be applied to the reaction involving nucleic acids.

3. The method for nucleic acid isolation according to claim 1, comprising adding a component in a manner such that the component mixed with the washing reagent remaining in the solid phase results in the reaction solution that can be applied to a reaction involving nucleic acids following elution of nucleic acids in the elution solution.

4. The method for nucleic acid isolation according to claim 1, wherein the washing reagent contains a salt and/or a surfactant.

5. The method for nucleic acid isolation according to claim 1, wherein a certain amount of the washing reagent is allowed to remain by adjusting the amount of the washing reagent to be added and the amount of the same to be discarded.

6. The method for nucleic acid isolation according to claim 1, wherein a certain amount of the washing reagent is allowed to remain by adjusting the moisture content of the solid phase and the moisture content of a solid-phase-supporting member that positions the solid phase.

7. The method for nucleic acid isolation according to claim 1, wherein a certain amount of the washing reagent is allowed to remain by adjusting discharge pressure or centrifugal force upon separation of the washing reagent from the solid phase.

8. The method for nucleic acid isolation according to claim 1, wherein the reaction is a polymerase chain reaction (PCR).

9. The method for nucleic acid isolation according to claim 1, wherein the reaction corresponds to the LAMP (loop-mediated isothermal amplification) method.

10. The method for nucleic acid isolation according to claim 1, wherein the washing reagent comprises at least one component selected from the group consisting of potassium chloride (KCI), magnesium chloride (MgCl₂), ammonium sulfate ((NH₄)₂SO₄), magnesium sulfate (MgSO₄), Tris-HCl, and polyoxyethylene sorbitan monolaurate.

11. The method for nucleic acid isolation according to claim 1, further comprising a treatment of allowing the nucleic acids to become adsorbed on a solid phase by allowing a solution containing nucleic acids to come into contact with the solid phase.

12. An instrument for nucleic acid isolation, comprising:
a supply unit for supplying a washing reagent that supplies a washing reagent comprising at least one component constituting a reaction solution that can be applied to a reaction involving nucleic acids to a solid phase capable of adsorbing nucleic acids;
a separation unit for separating a washing reagent that separates the solution from the solid phase in a manner such that a certain amount of the washing reagent supplied to the solid phase is allowed to remain in the solid phase; and
a supply unit for supplying an elution reagent that supplies an elution reagent to the nucleic-acid-adsorbing solid phase following removal of the washing reagent by the separation unit for separating a washing reagent; wherein
nucleic acids are isolated in an elution reagent supplied by the supply unit for supplying an elution reagent.

13. The instrument for nucleic acid isolation according to claim 12, wherein the elution reagent mixed with the washing reagent remaining in the solid phase has a composition that results in the reaction solution that can be applied to the reaction involving nucleic acids.

14. The instrument for nucleic acid isolation according to claim 12, further comprising an addition unit for adding a component that adds a component in a manner such that the component mixed with the washing reagent remaining in the solid phase results in the reaction solution that can be applied to a reaction involving nucleic acids following elution of nucleic acids in the elution solution.

15. The instrument for nucleic acid isolation according to claim 12, wherein the supply unit for supplying a washing reagent fills a container that accommodates the solid phase with a washing reagent.

16. The instrument for nucleic acid isolation according to claim 12, wherein the separation unit for separating a washing reagent removes a washing reagent with which a container that accommodates the solid phase is filled.

17. The instrument for nucleic acid isolation according to claim 12, wherein the supply unit for supplying an elution reagent fills a container that accommodates the solid phase with an elution reagent.

18. The instrument for nucleic acid isolation according to claim 12, wherein the solid phase is maintained in a container, a washing reagent is aspirated into the container by the supply unit for supplying a washing reagent, the washing reagent in the container is discharged by the separation unit for separating a washing reagent, and an elution reagent is aspirated into the container by the supply unit for supplying an elution reagent.

19. The instrument for nucleic acid isolation according to claim 18, wherein the container comprises a solid-phase-supporting member that positions the solid phase and a certain amount of a washing reagent is maintained in the solid-phase-supporting member and/or the solid phase.
